# EUROPEAN PATENT SPECIFICATION

(11) **EP 1 610 843 B1**
(45) Date of publication and mention of the grant of the patent: **11.03.2009**
(21) Application number: 04726351.2
(22) Date of filing: 07.04.2004
(51) Int. Cl.: A61M 1/16, B01F 1/00, B01J 4/00

(54) **Medical Cartridge with Filter-Slits**
Medizinische Kartusche mit Filter-Schlitzen
Cartouche médicale avec filtre comportant des fentes

(30) Priority: 07.04.2003 SE 0301022; 07.04.2003 US 461658 P
(43) Date of publication of application: 04.01.2006
(73) Proprietor: Gambro Lundia AB, 220 10 Lund (SE)
(72) Inventor: LORENTZON, Jan-Olof, S-380 30 Rockneby (SE); LOSELL, Ingvar, S-245 42 Staffanstorp (SE); TRYGGVASON, Ragnar, S-240 21 Löddeköpinge (SE)
(74) Representative: Berglund, Stefan
(86) International application number: PCT/SE2004/000558
(87) International publication number: WO 2004/089443

(56) References cited:
- EP-A2- 0 439 793
- WO-A1-97/29796
- US-A- 4 734 198

## Description

### BACKGROUND OF THE INVENTION AND PRIOR ART

The present invention generally relates to a preparation of a liquid solution intended for a medical procedure, in particular to on-line preparation of a sodium chloride or sodium bicarbonate solution for hemodialysis. The on-line preparation in view takes place in a cartridge containing a particulate or granular material, such as sodium bicarbonate or sodium chloride.

More specifically, the present invention relates to a cartridge for preparing a liquid solution for a medical procedure according to the pre-characterising portion of claim 1.

Further, the present invention relates to a use of a device in a cartridge for preparing a liquid solution for a medical procedure and arranged to contain a particulate material.

The present invention also refers to a system for preparing a liquid solution for a medical procedure according to the pre-characterising portion of claim 26.

Dialysis is a well-known method for treatment of kidney insufficiency. In hemodialysis, the blood of a patient suffering from impaired kidney function is conducted from a patient blood vessel to a dialysis machine and is returned to the patient after the treatment. The blood is conducted along one side of a permeable membrane in a dialyser or filter connected to the dialysis machine, at the same time as dialysis liquid or dialysate may be conducted along the opposite side of the same membrane. Waste substances or poisons are removed from the blood by means of diffusion and/or convection from the blood to the dialysis liquid through the membrane. Excess water is also removed from the blood.

On-line preparation of dialysis liquid is known. For example, on line preparation of a saturated bicarbonate solution from particulate bicarbonate contained in a cartridge is disclosed in DE-A-19801107.

Also EP-B-278100 discloses such a cartridge. The cartridge comprises a closed vessel provided with penetrateable membranes at its upper inlet end and its lower outlet end, respectively. Within the vessel, there is provided a bed of particulate material of sufficient quantity so as to be suitable for one dialysis treatment session. For instance, in connection with the preparation of a dialysis liquid, the concentrate may consist of particulate sodium chloride or sodium bicarbonate. The quantity thereof contained in the cartridge may be of the order of magnitude of 400-1500 grams.

In use, such a cartridge is first primed with a liquid, such as water, either from the top or from the bottom. Enough liquid is introduced into the cartridge during priming so that the liquid level is above the level of the particulate material. The particulate material is dissolved in the liquid and a saturated solution can leave the cartridge through the outlet in the bottom of the cartridge. As the solution leaves the cartridge a corresponding amount of new liquid is introduced into the cartridge. To establish that the degree of saturation of the solution leaving the cartridge is satisfactory, the conductivity of the solution may be measured. If an unsatisfactory saturation is detected by sensing the conductivity of the solution, an alarm in the dialysis machine may be triggered.

EP-A-439 793 discloses a cartridge for preparing a liquid solution for a medical procedure and arranged to contain a particulate material. The cartridge includes an inner space for housing the particulate material, an inlet arranged to permit the introduction of a liquid into the inner space and an outlet arranged to permit the discharge of liquid from the inner space. A device comprising a hollow body, defined by a wall enclosing a cavity of the body, is disclosed. The body has a centre axis, a first end and a second end. The first end is attached to the cartridge at the inlet in such a manner that the body extends into the inner space and the second end is located in the inner space of the cartridge. The first end is open and adapted for receiving the liquid to be introduced into the cartridge. The hollow body has one opening through the wall. The design of the opening is not described.

The above mentioned way of on-line preparation of liquid solutions for medical use by means of a cartridge containing particulate material has many advantages. There are, however, some disadvantages with the prior art cartridges. After a few hours of use, the mixture of the liquid and the particulate material in the cartridge may become inhomogenous. In some instances, clods may be formed. Moreover, in use of the cartridge, gas bubbles containing carbon dioxide may be formed. The clods may prevent such gas bubbles from rising to the liquid surface. When a bubble then is released, a channel may be created in the particulate materiel, which some times may extend all the way down to the lower outlet of the cartridge. This may allow unsaturated solution to leave the cartridge causing a conductivity alarm. The alarm must be taken care of either by the patient or by a nurse or medical attendant, If these problems occur, they may be remedied by knocking on the cartridge wall or by shaking the cartridge. However, the cartridge often has to be discarded, while still containing a considerable amount of particulate material, and replaced by a new cartridge. The changing of a cartridge is time-consuming and costly.

The formation of channels in the particulate material might have other causes. For example, the liquid may be introduced gradually through the top inlet and fall in drops down to and impact the liquid surface. This may give rise to pressure waves in the liquid, which in turn create liquid currents perpendicular to the surface of the bed of particulate material. These currents may work their way down in the bed of particulate material, creating channels in the particulate material reaching the lower outlet making it possible for unsaturated solution to leave the cartridge.

Another problem with the cartridges used up to now is the risk that the particulate material can escape via the inlet and the outlet. In order to reduce this risk, a felt pad or any similar porous member is incorporated in the inlet and the outlet. In particular, during the priming of the cartridge form below, i.e. via the outlet, the porous felt pad at the inlet is important. The porous felt pad is normally manufactured in another material than the rest of the cartridge, which is disadvantageous form a recycling point of view.

A further problem in connection with such cartridge is the potential risk of leakage of the particulate material before the cartridge is taken into use. Today this problem has been solved by arranging a closing mechanism over the inlet and outlet of the cartridge. Before use and connection to the dialysis machine of the cartridge, the closing mechanism has to be open. The closing mechanism may be, for instance, in the form of a valve, a removable cap or a solid cover to be broken by means of a sharp tool.

### SUMMARY OF THE INVENTION

The object of the present invention is to enable an improvement of the preparation of a liquid solution for a medical procedure, in particular a sodium chloride or bicarbonate solution for dialysis treatment, such as hemodialysis HD, hemofiltration HF or hemodiafiltration HDF among others. More specifically, the object is to provide a proper distribution of a liquid in a cartridge containing a particulate material. A further object is to prevent the particulate material from leaving the cartridge in an undesired manner.

This object is achieved by the cartridge as defined in claim 1.

By means of such a cartridge with particulate material, the liquid continuously introduced in the cartridge may be distributed in a desired way so as to minimize the risk of formation of channels and inhomogeneities in the particulate material. In turn this minimises the risk of unsaturated solution leaving the cartridge. The chance that the whole amount of particulate material in the cartridge can be used is thus increased. Moreover, during the initial priming of the cartridge, the device provides a more even distribution of the liquid. Due to the thin slit-shaped openings a desired pressure drop over the wall of the hollow body may be achieved. The desired pressure drop may be chosen so as to reduce the risks for channelling. The total flow area of the slit-shaped openings governing the speed of the supply of liquid may in a convenient manner be adjusted by the selection of an appropriate number of slit-shaped openings.

Furthermore, thanks to the thin slit-shaped openings any particles of the particulate material having a minimum size larger than the second extension will be efficiently prevented from leaving the cartridge via the inlet, especially before the device is used. This permits a simple and inexpensive handling of the cartridge before the actual use, for instance by means of the provision of an easily removable cap for closing the inlet before the device is to be used. Moreover, the previously used felt pad may be dispensed with.

According to an embodiment of the present invention, said hollow body has a centre axis and an elongated, tubular shape along the centre axis. By means of such an elongated shape, it is possible to let the liquid be output below the liquid/air surface also when the liquid level is sinking gradually. Therefore, the liquid will not fall in drops down onto the liquid surface. The generation of pressure waves is thus avoided. Consequently, the formation of channels in the particulate material is minimised.

According to a further embodiment of the present invention, said hollow body is tapering along the centre axis towards the second end. By means of such tapering shape, the device can easily be stuck into the dry particulate material when the cartridge is prepared.

According to a further embodiment of the present invention, said hollow body at the first end has engaging means adapted for connection of the device to the cartridge.

According to a further embodiment of the present invention, the second extension is significantly shorter than the length of the slit-shaped opening in the flow direction. This feature permits a certain wall thickness of the body of the device ensuring a sufficient strength of the device. Preferably, the first extension is substantially perpendicular to the flow direction.

According to a further embodiment of the present invention, the second extension is equal to or less than 0,1 mm, preferably equal to or less than 0,08 mm. Moreover, the second extension may be equal to or more than 0,02 mm, preferably equal to or more than 0,04 mm. According to an advantageous embodiment, the second extension is approximately 0,06 mm.

According to a further embodiment of the present invention, said slit-shaped openings are distributed around the wall. In such way, the liquid supply into the particulate material may be uniformly distributed. It is to be noted that the hollow body may have any cross-sectional shape, such as a circular, oval, triangular, square, rectangular or any other polygonal shape. In case of a rectangular or square cross-sectional shape, an appropriate liquid distribution may be obtained even if slit-shaped openings are arranged through only one or two of the side walls of such a shape.

According to a further embodiment of the present invention, said hollow body has a wall portion at least in the proximity of the second end, and wherein said slit-shaped opening extend through said wall portion. Such a wall portion at the second lower end permits the slit-shaped opening or openings at least initially to be located beneath the upper liquid surface. However, such a wall portion is useful even if the liquid surface is located below the wall portion.

According to a further embodiment of the present invention, said wall portion has a tip-like shape, especially said wall portion may be substantially conical.

According to further embodiment of the present invention, said wall portion is substantially plane. Such a substantially plane wall portion is advantageous from a manufacturing point of view, since it permits a relatively simple injection moulding process. According to one alternative, a normal direction of said wall portion may form an angle of inclination to the centre axis, wherein the flow direction forms an angle to the centre axis. According to another alternative, the centre axis extends substantially in parallel with a normal direction of the plane.

According to a further embodiment of the present invention, the slit-shaped opening has an upstream end and a downstream end with respect to the flow direction, wherein the second extension of the slit-shaped opening increases in the flow direction from a minimum value at the upstream end of the slit-shaped opening to a maximum value at the downstream end of the opening. Such a shape of the slit-shape opening or openings is also advantageous from a manufacturing point of view.

According to an advantageous embodiment, the cartridge may thus be provided with a device provided at the inlet and having at least one thin slit-shaped opening, and a filter provided at the outlet and having at least one slit-shaped opening. By such a cartridge, the particulate material in the solid state may be securely maintained in the cartridge.

The object is also achieved by a use of a cartridge as defined in claim 22. Advantageous embodiments of the use are defined in the dependent claims 23 to 25.

The object is also achieved by the system as defined in claim 26. Advantageous embodiments of the system are defined in the dependent claims 27 to 31.

### BRIEF DESCRIPTION OF THE DRAWINGS

The present invention is now to be described more closely by the following description of various embodiments and with reference to the drawings attached.
- Fig 1: discloses schematically a system for preparing a liquid solution for a medical procedure.
- Fig 2: discloses schematically a cartridge including a device according the present invention.
- Fig 3: discloses schematically a plan view of a filter to be arranged in the cartridge of Fig 2.
- Fig 4: discloses schematically a sectional view of the filter along the line IV-IV in Fig 3.
- Fig 5: discloses schematically a sectional view of the filter along the line V-V in Fig 3.
- Fig 6: discloses schematically a longitudinal sectional view of a part of the device arranged in the cartridge of Fig 2.
- Fig 7: discloses schematically a transversal sectional view of the part of the device arranged in the cartridge of Fig 2.
- Fig 8: discloses schematically a perspective view of a first embodiment of the device arranged in the cartridge of Fig 2.
- Fig 9: discloses schematically a perspective view of a second embodiment of a device to be arranged in the cartridge of Fig 2.
- Fig 10: discloses schematically a perspective view of a third embodiment of a device to be arranged in the cartridge of Fig 2.
- Fig 11: discloses schematically a perspective view of a fourth embodiment of a device to be arranged in the cartridge of Fig 2.

### DETAILED DESCRIPTION OF VARIOUS EMBODIMENTS OF THE INVENTION

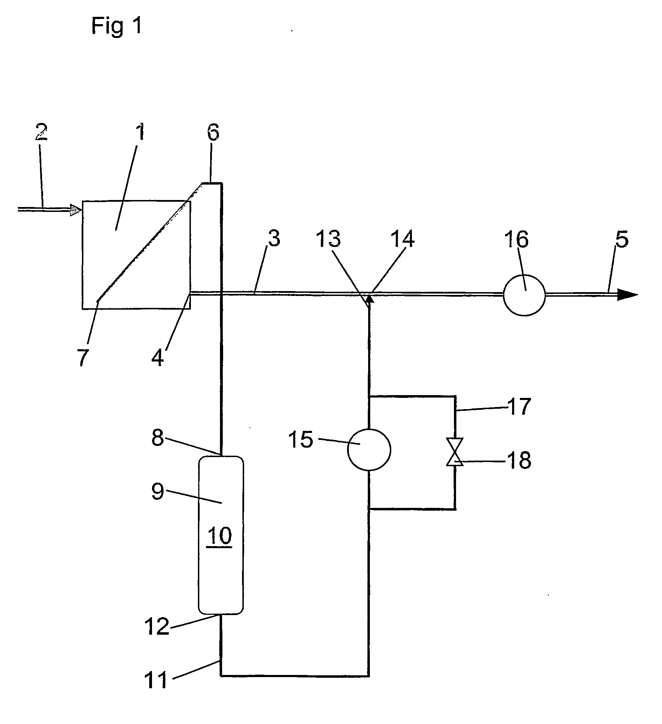

Fig 1 discloses schematically a system for preparing a liquid solution for a medical procedure. In particular, the system is designed for preparing a dialysis liquid solution for the performance of a hemodialysis treatment. The system may also be used for hemodiafiltration and hemofiltration. The system disclosed in Fig 1 may thus form a part of a dialysis equipment.

The system includes a source 1 containing a liquid, and in particular a dialysis liquid. The source 1 may be supplied with the liquid via an inlet conduit 2. A first liquid conduit 3 has a first end 4 communicating with the source 1 to withdraw the dialysis liquid into the first liquid conduit 3. The first liquid conduit 3 also has a second end 5 for delivering a dialysis liquid solution to a receiver (not disclosed) such as a dialysis equipment. A second liquid conduit 6 has a first end 7 communicating with the source 1 and a second end 8 communicating with the inlet of a cartridge 9 for the introduction of the liquid from the source 1 into an inner space 10 of the cartridge 9 to produce a concentrate liquid solution. A third liquid conduit 11 has a first end 12 communicating with the outlet of the cartridge 9 and a second end 13 communicating with the first liquid conduit 3 at a mixing point 14 intermediate said first end 4 and said second end 5.

The third liquid conduit 11 is thus arranged to withdraw said concentrate liquid solution from the cartridge 9 into the first liquid conduit 3 to be mixed with the liquid conducted through the first liquid conduit 3 from the source 1 in order to produce a liquid solution for delivery to said receiver. The concentrate liquid solution is transported through the third liquid conduit 11 by means of a pump 15. The liquid is transported from the source 1 to said receiver through the first liquid conduit 3 by means of a pump 16. A control loop 17 including a valve 18 may be arranged to control the quantity of concentrate liquid solution to be delivered to the liquid of the first liquid conduit 3.

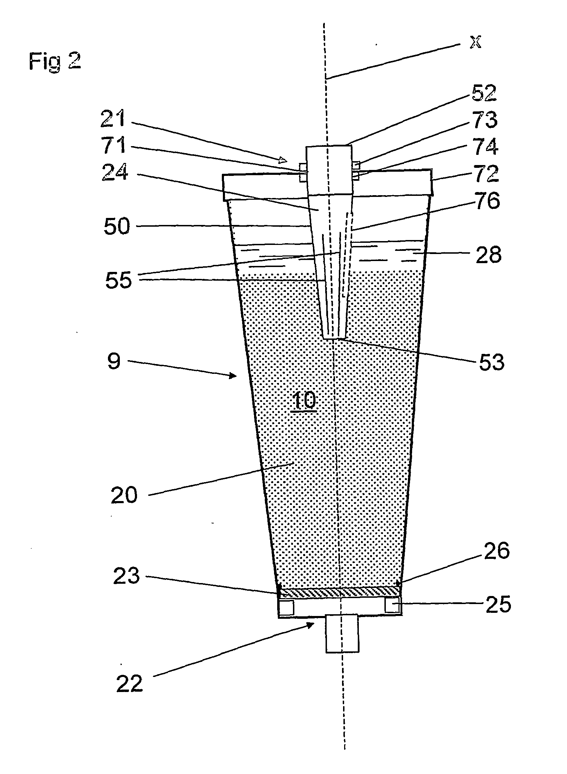

The cartridge 9 is explained below with reference to Fig 2. In the embodiment disclosed, it is referred to the production of a dialysis liquid solution to which sodium bicarbonate has been added. It is to be noted that also other substances than sodium bicarbonate can be added in a similar manner to the dialysis liquid, for instance sodium chloride and other salts. It is also to be noted, that the system, the cartridge and the components included therein can be used also for producing other liquid solutions than a dialysis liquid.

The cartridge 9 is arranged to contain a particulate material 20 in the inner space 10. In the embodiment disclosed the particulate material 20 is a sodium bicarbonate powder, The cartridge 9 has an inlet 21 which is arranged to permit the introduction of a liquid into the inner space 10. In the embodiment disclosed, the inlet 21 is connected to and communicates with the second liquid conduit 6.

The cartridge 9 also has an outlet 22 arranged to permit the discharge of liquid from the inner space 10. In the embodiment disclosed, the outlet 22 is connected to and communicates with the third liquid conduit 11 for discharging the concentrate liquid solution. The cartridge disclosed in Fig 2 has been prepared for use. In a first step, air has been sucked from the cartridge 9 for the purpose of creating a vacuum in the cartridge 9. Then the cartridge 9 has been primed with a liquid 28, i.e. a solvent, such as water, by letting the liquid 28 flow into the cartridge 9. As appears from Fig 2, the liquid 28 forms a liquid layer, wherein the level of the liquid 28 is above the level of the particulate material 20.

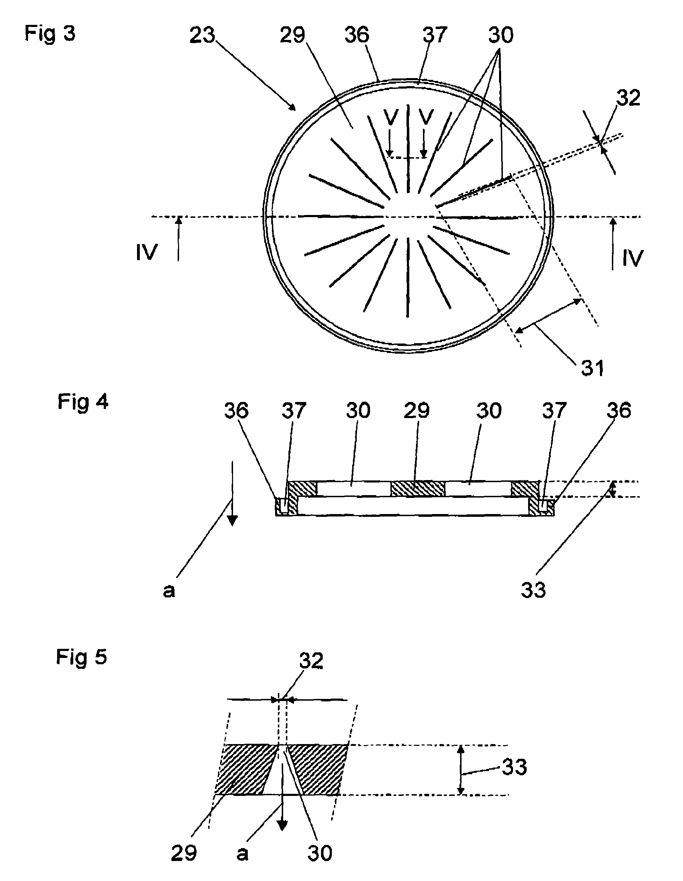

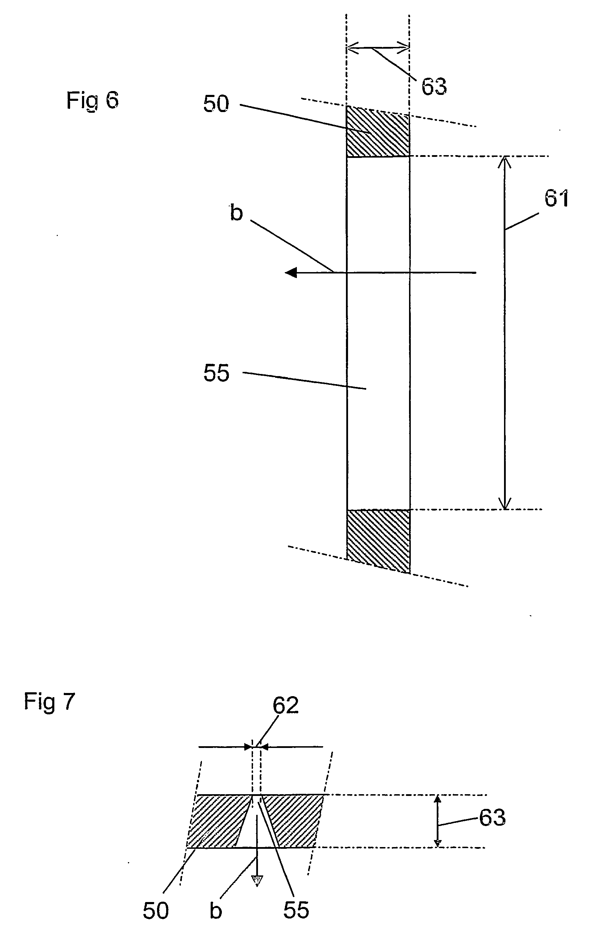

A filter 23 is arranged at the outlet 22 to permit passage of said liquid through the filter 23, but to prevent the passage of the particulate material 20 through the filter 23. The filter 23 defines a filter direction a, see Figs 4 and 5, and thus permits the liquid to pass through the filter 23 in the filter direction a. The cartridge 9 also includes a device in the form of an elongated hollow body 24 arranged at the inlet 21 to permit passage of the liquid through the hollow body 24, but to prevent passage of the particulate material 20 through the hollow body 24. The hollow body 24 has a centre axis x, extending substantially vertically during normal use of the device and the cartridge 9. The centre axis x also forms a centre axis x for the cartridge 9. The hollow body 24 defines a flow direction b, see Figs 6 and 7, and permits the liquid to pass through the hollow body 24 in the flow direction b into the inner space 10.

The filter 23 rest on a support member in the form of a shoulder 25 extending around the inner periphery of the inner space 10 in the proximity of the outlet 22. A smaller shoulder 26 extends around the inner periphery of the inner space 10 above the filter 23 in order to permit the locking of the filter 23 at its position on the shoulder 25. Two different embodiments of the filter 23 are explained below with reference to Figs 3-7.

Figs 3-5 disclose an embodiment of the filter 23. The filter 23 has the shape of a substantially circular and substantially plane disc 29 having a main extension plane. It is to be noted, that the thickness of the disc 29 is exaggerated in the figures in order to simplify the explanation of the filter construction. The filter 23 includes a number of slif-shaped openings 30. Each such opening 30 has a first extension, Indicated by the line 31 in Fig 3 for one of the openings 30. Each opening 30 also has a second extension, indicated by the line 32 in Figs 3 and 5. The second extension 32 is substantially perpendicular to the first extension 31. Both the second extension 32 and the first extension 31 are substantially perpendicular to the filter direction a.

The second extension 32 is significantly shorter than the first extension 31. Moreover, the second extension 32 is also significantly shorter than the length 33 of the slit-shaped opening 30 in the filter direction a. For instance the length 33 may be 10 - 1000 times the second extension 32. In particular, the second extension 32 is equal to or less than 0,1 mm, preferably equal to or less than 0,08 mm. Moreover, the second extension 32 is equal to or more than 0,02 mm, preferably equal to or more than 0,04 mm. According to a preferred embodiment, the second extension 32 is approximately 0,06 mm.

The slit-shaped openings 30, which extend through the disc 29, are, in the embodiment disclosed, provided to extend in a radial direction with regard to the first extension 31 and a centre point of the disc 29. However, the slit-shaped openings 30 may also be arranged to extend in other directions, for instance the disc 29 may include a plurality of slit-shaped openings 30 extending in parallel to each other. The disc 29 is made in a polymer material, for instance polycarbonate or polypropylene. The disc 29 may be manufactured through an injection moulding process by injecting said polymer material into a mould cavity having the shape of the disc 29. Each slit-shaped opening 30 has an upstream end and a downstream end with respect to the filter direction a. As appears from Fig 5, the second extension 32 of the slit-shaped opening 30 increases in the filter direction a from a minimum value at the upstream end of the slit-shaped opening 30 to a maximum value at the downstream end of the slit-shaped opening 30. Such a shape of the slit-shaped opening facilitates the injection moulding of the disc 29.

The filter 23 includes a circumferential flexible edge portion 36 formed as a part of the disc 29. The edge portion 36 is flexible in an radial inward direction and formed by the provision of a circumferential groove 37 extending radially inside the edge portion 36. Consequently, the flexible edge portion 36 may be bent radially inwardly when the filter 23 is introduced at its position on the shoulder 25 in order to pass the shoulder 26. After having passed the shoulder 26, the flexible edge portion 36 may flex back to the outer position disclosed in Fig 4.

The device in the form of the hollow body 24, and its arrangement in the cartridge 9, is now to be explained more closely with reference to Figs 2 and 6 to 11, disclosing different embodiments of the hollow body 24. The hollow body 24 has an elongated extension along the centre axis x. Furthermore, the hollow body 24 may have tubular shape along the centre axis x, especially a tapering shape along the centre axis x towards the second end 53. In the embodiments disclosed, a main part of the hollow body 24 is substantially conical. The hollow body 24 is defined by a wall 50 enclosing a cavity of the body 24. The hollow body 24 has a first end 52, forming an upper end during normal use of the hollow body 24, and a second end 53, forming a lower end during normal use of the hollow body 24. The hollow body 24 is provided with a plurality of slit-shaped openings 55 extending through the wall 50.

The second end 53 is in some of the embodiments closed. The first end 52 is open and adapted for receiving the liquid to be introduced into the cartridge 9. The liquid leaves the cavity of the hollow body 24 through the slit-shaped openings 55 in a flow direction b. Each such slit-shaped opening 55 defines a specific, respective flow direction b, see Figs 6 and 7. The hollow body extends downwardly into the inner space 10 of the cartridge 9. At least initially, the hollow body 24 extends down to the liquid 28 and the particulate material 20, so that the liquid supplied via the hollow body 24 may flow substantially horizontally into the bed of particulate material 2Q, or at least into the liquid 28.

In the same manner as for the filter 23, each slit-shaped opening 55 has a first extension, indicated by the line 61 in Fig 6, and a second extension, indicated by the line 62 in Fig 7. The second extension 62 is substantially perpendicular to the first extension 61. Both the second extension 62 and the first extension 61 are substantially perpendicular to the flow direction b.

The second extension 62 is significantly shorter than the first extension 61. Moreover, the second extension 62 is significantly shorter than the length 63 of the slit-shaped opening 55 in the flow direction b. In particular, the second extension 62 is equal to or less than 0,1 mm, preferably equal to or less than 0,08 mm. Moreover, the second extension 62 is equal to or more than 0,02 mm, preferably equal to or more than 0,04 mm. According to a preferred embodiment, the second extension 62 is approximately 0,06 mm.

The hollow body 24 is made in a polymer material, for instance polycarbonate or polypropylene. The hollow body 24 may be manufactured through an injection moulding process by injecting said polymer material into a mould cavity having the shape of the hollow body 24. Each slit-shaped opening 55 has an upstream end and a downstream end with respect to the respective flow direction b. As appears from Fig 7, the second extension 62 of the slit-shaped opening 55 increases in the flow direction b from a minimum value at the upstream end of the slit-shaped opening 55 to a maximum value at the downstream end of the slit-shaped opening 55. Such a shape of the slit-shaped openings 55 facilitates the injection moulding process of the hollow body 24.

The hollow body 24 also has an engagement portion 71 at the first, upper end 52 to be engaged by an upper portion of the cartridge 9, This upper portion may be formed by an attachable lid 72. The upper portion has an aperture into which the hollow body is introduced. The engagement portion 71 includes two flanges 73 and 74 extending around the outer periphery of the hollow body 24 and provided successively after each other along the centre axis x. When introduced into the aperture of the upper portion, the flanges 73, 74 are arranged on a respective side of the wall of the upper portion or the lid 72 as appears from Fig 2. Alternatively, the hollow body 24 may be manufactured in one single piece with the upper portion or the lid 72.

The hollow body 24 may also be provided with one or several elongated flanges or ridges 76. One such flange 76 is disclosed by dotted lines in Fig 2. The flanges 76 may be arranged longitudinally along the inside wall of the hollow body 24. The flanges 76 may act as "liquid guides". The incoming liquid may then follow the inside wall of the hollow body 24 or flow along the flanges 76. Alternatively, the liquid guides may include plastic pellets.

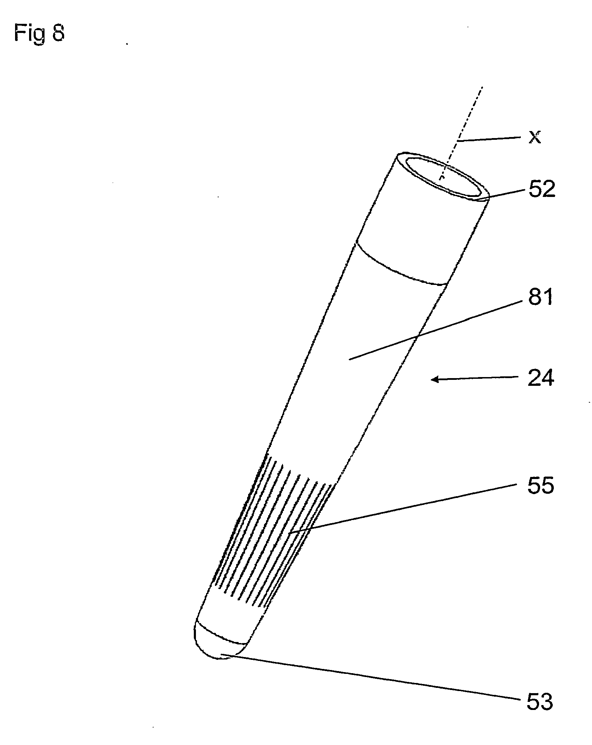

In the embodiment disclosed in Fig 8, the hollow body 24 has a main wall portion 81 which has a substantially conical shape. In the proximity of the second lower end 53, the conical wall portion 81 of the hollow body 24 is provided with a plurality of slit-shaped openings 55 uniformly distributed around the hollow body 24. The first extension 61 of each slit-shaped opening 55 has a first main component extending in parallel with the centre axis x, and, due to the conical shape of the wall portion 81, a second minor component extending substantially radially outwardly from the centre axis x. The flow direction b may thus extend outwardly with a substantially right angle to the centre axis x. It is to be noted that the hollow body 24 may also have another cross-sectional shape than a circular one, for instance oval, triangular, square, rectangular or any other polygonal shape. In case of any polygonal shape, all or a selected number of the side walls so formed may be provided with slit-shaped openings 55. In particular, it may be mentioned that if the hollow body 24 has a square or rectangular cross-sectional shape only one or two of the side walls may be provided with slit-shaped openings 55.

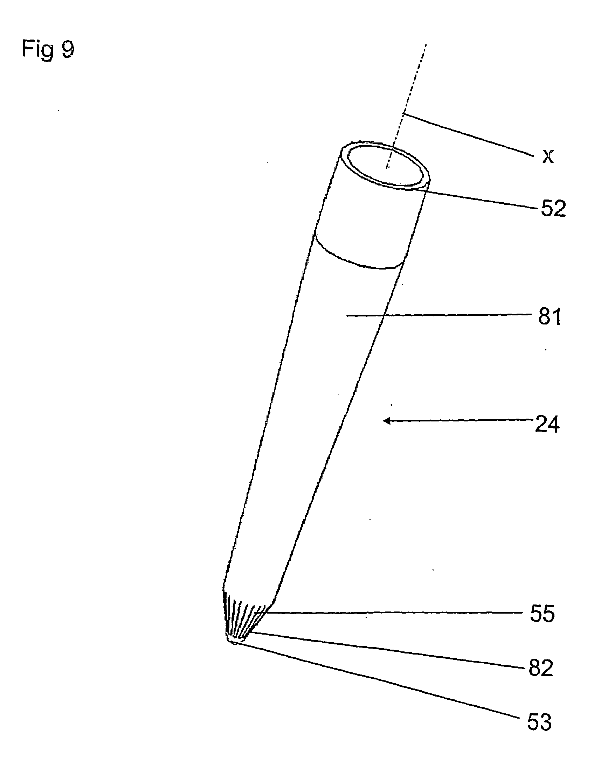

In the embodiment disclosed in Fig 9, the hollow body 24 also has a main wall portion 81 which has a substantially conical shape. In addition, at the second lower end 53, the hollow body 24 has a smaller wall portion 82, which has a tip-like shape, and more precisely a substantially conical shape. The cone angle with respect to the centre axis x is significantly greater for the smaller wall portion 82 than for the major wall portion 81. The smaller conical wall portion 82 is provided with a plurality of slit-shaped openings 55 uniformly distributed around the hollow body 24. The first extension 61 of each slit-shaped opening 55 has a first component extending in parallel with the centre axis x and second component extending substantially radially outwardly from the centre axis x. Thus the flow direction b will form an angle to the centre axis x. It is to be noted that also in this embodiment, the hollow body and the smaller wall portion 82 may have another cross-sectional shape than a circular one, for instance oval, triangular, square, rectangular or any other polygonal shape. Also in this case the number of side walls provided with slit-shaped openings 55 may be selected in accordance with circumstances.

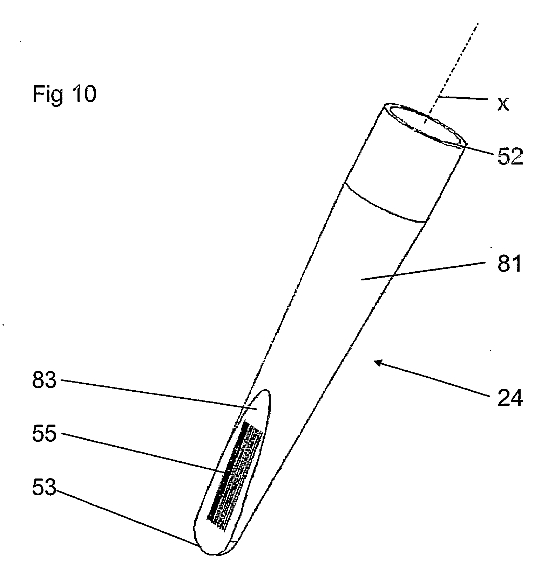

In the embodiment disclosed in Fig 10, the hollow body 24 also has a main wall portion 81 with a substantially conical shape. In addition, at the second lower end 53, the hollow body 24 has a smaller wall portion 83, which has a substantially plane shape. The wall portion 83 is positioned in such a way that the plane wall portion 83 is sloping and that a normal direction of the wall portion 83 and forms an angle of inclination to the centre axis x. In the embodiment disclosed, this angle is close to 90°. The plane wall portion 83 is provided with a plurality of slit-shaped openings 55 extending in parallel to each other. The first extension 61 of each slit-shaped opening 55 has a first component extending in parallel with the centre axis x. Also in this case, the flow direction b will form an angle to the centre axis x, which is close to 90°.

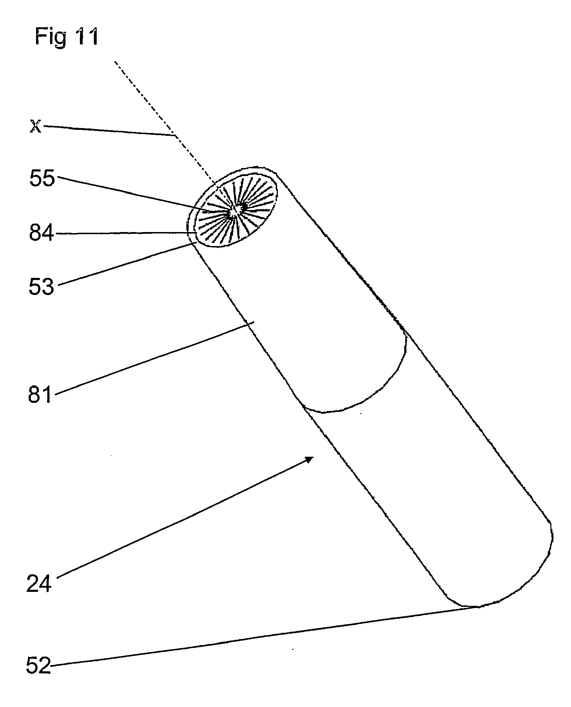

In the embodiment disclosed in Fig 11, the hollow body 24 also has a main wall portion 81 with a substantially conical shape. In addition, at the second lower end 53, the hollow body 24 has a smaller transverse wall portion 84, which has a substantially plane shape. The transverse plane wall portion 84 is positioned in such a way that a normal direction of the wall portion 84 is parallel with the centre axis x. The transverse plane wall portion 84 is provided with a plurality of slit-shaped openings 55 extending in a radial direction with respect to the centre axis x. The layout of the slit-shaped openings 55 of the transverse wall portion 84 is similar to the layout of the slit-shaped openings 30 of the disc 29 of the filter 23.

By means of the hollow body 24 for the supply of liquid to the particulate material 20, the liquid will be introduced directly into the particulate material 20 or the liquid layer 28. The liquid may then be prevented from freely falling in drops down to the liquid surface. The pressure waves and the currents will then be directed towards the inside wall of the cartridge 9 and dampened out before reaching the particulate material 20. The channelling effect may thus be minimised. According to the embodiments of Figs 8 to 10, the liquid may also be introduced substantially horizontally into the particulate material 20 or into the liquid layer 28. Tests have shown that the functioning time before an alarm is triggered can be prolonged as much as two hours compared to when using a prior art cartridge, intended for example for 8 hours of use.

The present invention is not limited to the embodiments disclosed but may be varied and modified within the scope of the following claims. In the embodiments disclosed the first extension 31, 61 of the filter 23 and the hollow body 24 is substantially straight. It is to be noted, however, that the first extension 31, 61 as an alternative may be curved.

## Claims

1. A cartridge for preparing a liquid solution for a medical procedure and arranged to contain a particulate material (20), wherein the cartridge (9) includes:
an inner space (10) for housing the particulate material (20);
an inlet (21) arranged to permit the introduction of a liquid into the inner space (10);
an outlet (22) arranged to permit the discharge of liquid from the inner space (10); and
a device comprising a hollow body (24) defined by a wall (50) enclosing a cavity of the body, the body having a centre axis (x), a first end (52) and a second end (53), said first end being mounted to the cartridge (9) at the inlet in such a manner that the body (24) extends into the inner space (10) and said second end is located in the inner space of the cartridge, said first end being open and adapted for receiving said liquid to be introduced into the cartridge,
wherein the device includes a plurality of slit-shaped openings (55), which extend through the wall (50), wherein the liquid leaves the device through said slit-shaped openings (55) in a flow direction (b), wherein said slit-shaped openings have a first extension (61) and a second extension (62) being substantially perpendicular to the flow direction (b) and to the first extension (61), wherein the second extension (62) is significantly shorter than the first extension (61) and significantly shorter than the length (63) of the slit-shaped openings (55) in the flow direction (b).

2. A cartridge according to claim 1, wherein said hollow body (24) has an elongated, tubular shape along the centre axis (x).

3. A cartridge according to any one of claims 1 and 2, wherein said hollow body (24) is tapering along the centre axis (x) towards the second end (53).

4. A cartridge according to any one of the preceding claims, wherein said hollow body (24) at the first end (52) has engaging means (73, 74) adapted for connection of the device to the cartridge (9).

5. A cartridge according to any one of the preceding claims, wherein the first extension (61) is substantially perpendicular to the flow direction (b).

6. A cartridge according to any one of the preceding claims, wherein the second extension (62) is equal to or less than 0,1 mm.

7. A cartridge according to any one of the preceding claims, wherein the second extension (62) is equal to or less than 0,08 mm.

8. A cartridge according to any one of the preceding claims, wherein the second extension (62) is equal to or more than 0,02 mm.

9. A cartridge according to any one of the preceding claims, wherein the second extension (62) is equal to or more than 0,04 mm.

10. A cartridge according to any one of the preceding claims, wherein the second extension (62) is approximately 0,06 mm.

11. A cartridge according to any one of the preceding claims, wherein said slit-shaped openings (55) are distributed around the wall (50).

12. A cartridge according to any one of the preceding claims, wherein said hollow body (24) has a wall portion (81-84) at least in the proximity of the second end (53), and wherein said slit-shaped opening (55) extend through said wall portion (81-84).

13. A cartridge according to claim 12, wherein said wall portion (82) has a tip-like shape.

14. A cartridge according to claim 13, wherein said wall portion (82) is substantially conical.

15. A cartridge according to claim 14, wherein said wall portion (83, 84) is substantially plane.

16. A cartridge according to claim 15, wherein a normal direction of said wall portion (83) forms an angle of inclination to the centre axis (x).

17. A cartridge according to any one of the preceding claims, wherein the flow direction (b) forms an angle to the centre axis (x).

18. A cartridge according to claim 17, wherein the centre axis (x) extends substantially in parallel with a normal direction of the plane wall portion (84).

19. A cartridge according to any one of the preceding claims, wherein the slit-shaped opening (55) has an upstream end and a downstream end with respect to the flow direction (b), wherein the second extension of the slit-shaped opening increases in the flow direction (b) from a minimum value at the upstream end of the slit-shaped opening to a maximum value at the downstream end of the opening (55).

20. A cartridge according to any one of claims 1 to 19, wherein the cartridge (9) includes a filter (23) arranged at the outlet (22) and to permit passage of the liquid through the filter (23), but to prevent passage of the particulate material through the filter, wherein the filter permits the liquid to pass through the filter in a filter direction (a).

21. A cartridge according to claim 20, wherein the filter (23) includes at least one slit-shaped opening (30), which has a first extension (31) and a second extension (32) being substantially perpendicular to the filter direction (a) and to the first extension, wherein the second extension is significantly shorter than the first extension.

22. A use of a device in a cartridge (9) according to any one of claims 1 to 21, for preparing a liquid solution for a medical procedure and arranged to contain a particulate material (20), the use including the step of supplying said liquid to the cartridge (9) via the inlet (21) in such a way that the liquid passes through the particulate material (20) and thereby dissolves at least a part of the particulate material to form a liquid solution.

23. A use according to claim 22, wherein the liquid is a dialysis liquid.

24. A use according to any one of claims 22 and 23, wherein the particulate material includes bicarbonate and/or sodium chloride.

25. A use according to any one of claims 22 to 24, wherein the device includes the features of any one of claims 2 to 19.

26. A system for preparing a liquid solution for a medical procedure, the system including:
a cartridge (9) containing a particulate material in an inner space thereof and including an inlet and an outlet;
a first liquid (3) conduit having a first end (4) communicating with a source (1) of liquid to withdraw the liquid into the first liquid conduit and a second end;
a second liquid conduit (6) having a first end (7) communicating with the source (1) of liquid and a second end (8) communicating with the inlet of the cartridge (9) for introducing the liquid into the inner space (10) to produce a concentrate liquid solution containing at least a part of the particulate material dissolved in the liquid; and a third liquid conduit (11) communicating with the outlet of the cartridge and with a mixing point (13) in the first liquid conduit (3) intermediate said first and second ends (4, 5) for conducting said concentrate liquid solution from the cartridge (9) into said first liquid conduit to be mixed with the liquid being conducted through the first liquid conduit to thereby produce said liquid solution in the first liquid conduit for delivery to said second end of the first liquid conduit, the Cartridge comprising a device as defined in any one of claims 1 to 19.

27. A system according to claim 26, wherein the device includes the features of any one of claims 2 to 19.

28. A system according to any one of claims 26 and 27, wherein the cartridge (9) includes filter (23) arranged at the outlet (22) and to permit passage of the liquid through the filter (23), but to prevent passage of the particulate material through the filter, wherein the filter permits the liquid to pass through the filter in a filter direction (a).

29. A system according to claim 28, wherein the filter includes at least one slit-shaped opening (30), which has a first extension (31) and a second extension (32) being substantially perpendicular to the filter direction (a) and to the first extension, wherein the second (32) extension is significantly shorter than the first extension (31).

30. A system according to any one of claims 26 to 29, wherein the liquid is a dialysis liquid.

31. A system according to any one of claims 26 to 30, wherein the particulate material includes bicarbonate and/or sodium chloride.

## Patentansprüche

1. Kartusche zur Vorbereitung einer flüssigen Lösung für ein medizinisches Verfahren und angeordnet zum Beeinhalten eines partikulären Materials (20), wobei die Kartusche (9) umfasst:
einen Innenraum (10) zur Beherbergung des partikulären Materials (20), einen Einlass (21), welcher angeordnet ist zum Erlauben des Einführens einer Flüssigkeit in den Innenraum (10),
einen Auslass (22), welcher angeordnet ist zum Erlauben des Ablassens der Flüssigkeit aus dem Innenraum (10) und
eine Vorrichtung, umfassend einen Hohlkörper (24), welcher durch eine Wand (50) definiert ist, die einen Hohlraum des Körpers umgibt, wobei der Körper eine Zentralachse (x), ein erstes Ende (52) und ein zweites Ende (53) aufweist, wobei das erste Ende auf der Kartusche (9) bei dem Einlass befestigt ist, so dass sich der Körper (24) in den Innenraum (10) erstreckt und sich das zweite Ende in dem Innenraum der Kartusche befindet, wobei das erste Ende offen ist, und angepasst zum Empfang der in die Kartusche einzufüllenden Flüssigkeit, wobei die Vorrichtung eine Vielzahl schlitzförmiger Öffnungen (55) umfasst, welche sich durch die Wand (50) erstrecken, wobei die Flüssigkeit die Vorrichtung durch die schlitzförmigen Öffnungen (55) in einer Flussrichtung (b) verlässt, wobei die schlitzförmigen Öffnungen eine erste Ausdehnung (61) und eine zweite Ausdehnung (62) aufweisen, welche im Wesentlichen senkrecht zu der Flussrichtung (b) und zu der ersten Ausdehnung (61) sind, wobei die zweite Ausdehnung (62) deutlich kürzer ist als die erste Ausdehnung (61) und deutlich kürzer als die Länge (63) der schlitzförmigen Öffnungen (55) in der Flussrichtung (b).

2. Kartusche nach Anspruch 1, wobei der Hohlkörper (24) eine gestreckte, röhrenförmige Form entlang der Zentralachse (x) aufweist.

3. Kartusche nach irgendeinem der Ansprüche 1 und 2, wobei sich der Hohlkörper (24) entlang der Zentralachse (x) in Richtung des zweiten Endes (53) verjüngt.

4. Kartusche nach irgendeinem der vorherigen Ansprüche, wobei der Hohlkörper (24) an dem ersten Ende (52) eingreifende Mittel (73, 74) aufweist, die angepasst sind zur Verbindung der Vorrichtung mit der Kartusche (9).

5. Kartusche nach irgendeinem der vorherigen Ansprüche, wobei die erste Ausdehnung (61) im Wesentlichen senkrecht zu der Flussrichtung (b) ist.

6. Kartusche nach irgendeinem der vorherigen Ansprüche, wobei die zweite Ausdehnung (62) gleich oder kleiner als 0,1 mm ist.

7. Kartusche nach irgendeinem der vorherigen Ansprüche, wobei die zweite Ausdehnung (62) gleich oder kleiner als 0,08 mm ist.

8. Kartusche nach irgendeinem der vorherigen Ansprüche, wobei die zweite Ausdehnung (62) gleich oder größer als 0,02 mm ist.

9. Kartusche nach irgendeinem der vorherigen Ansprüche, wobei die zweite Ausdehnung (62) gleich oder größer als 0,04 mm ist.

10. Kartusche nach irgendeinem der vorherigen Ansprüche, wobei die zweite Ausdehnung (62) ungefähr 0,06 mm beträgt.

11. Kartusche nach irgendeinem der vorherigen Ansprüche, wobei die schlitzförmigen Öffnungen (55) um die Wand (50) angeordnet sind.

12. Kartusche nach irgendeinem der vorherigen Ansprüche, wobei der Hohlkörper (24) einen Wandabschnitt (81 bis 84) zumindest in der Nähe des zweiten Endes (53) aufweist, und wobei sich die schlitzförmigen Öffnungen (55) durch den Wandabschnitt (81 bis 84) erstrecken.

13. Kartusche nach Anspruch 12, wobei der Wandabschnitt (82) eine düsenähnliche Form hat.

14. Kartusche nach Anspruch 13, wobei der Wandabschnitt (82) im Wesentlichen konisch ist.

15. Kartusche nach Anspruch 14, wobei der Wandabschnitt (83, 84) im Wesentlichen eben ist.

16. Kartusche nach Anspruch 15, wobei eine normale Richtung des Wandabschnitts (83) einen Neigungswinkel zu der Zentralachse (x) bildet.

17. Kartusche nach irgendeinem der vorherigen Ansprüche, wobei die Flussrichtung (b) einen Winkel mit der Zentralachse (x) bildet.

18. Kartusche nach Anspruch 17, wobei sich die Zentralachse (x) im Wesentlichen parallel zu einer normalen Richtung des ebenen Wandabschnitts (84) erstreckt.

19. Kartusche nach irgendeinem der vorherigen Ansprüche, wobei die schlitzförmigen Öffnungen (55) ein stromaufwärtiges und ein stromabwärtiges Ende in Bezug auf die Flussrichtung (b) aufweist, wobei die zweite Ausdehnung der schlitzförmigen Öffnung in der Flussrichtung (b) von einem Minimalwert an dem stromaufwärtigen Ende der schlitzförmigen Öffnung auf einem Maximalwert an dem stromabwärtigen Ende der Öffnung (55) zunimmt.

20. Kartusche nach irgendeinem der Ansprüche 1 bis 19, wobei die Kartusche (9) einen Filter (23) einschließt, welcher an dem Auslass (22) angeordnet ist, und einen Durchgang der Flüssigkeit durch den Filter (23) erlaubt, jedoch den Durchgang von partikulärem Material durch den Filter verhindert, wobei der Filter der Flüssigkeit erlaubt, durch den Filter in einer Filterrichtung (a) hindurchzugehen.

21. Kartusche nach Anspruch 20, wobei der Filter (23) zumindest eine schlitzförmige Öffnung (30) umfasst, welcher eine erste Ausdehnung (31) und eine zweite Ausdehnung (32) aufweist, die im Wesentlichen senkrecht zu der Filterrichtung (a) und zu der ersten Ausdehnung ist, und wobei die zweite Ausdehnung deutlich kürzer ist, als die erste Ausdehnung.

22. Verwendung einer Vorrichtung in einer Kartusche (9) nach irgendeinem der Ansprüche 1 bis 21 zur Vorbereitung einer flüssigen Lösung für ein medizinisches Verfahren und angeordnet zum Beeinhalten eines partikulären Materials (20), wobei die Verwendung den Schritt der Zufuhr der Flüssigkeit zu der Kartusche (9) über den Einlass (21) umfasst, in solch einer Art, dass die Flüssigkeit durch das partikuläre Material (20) hindurchfließt, und dabei zumindest einen Teil des partikulären Materials löst, um eine flüssige Lösung zu bilden.

23. Verwendung nach Anspruch 22, wobei die Flüssigkeit eine Dialyseflüssigkeit ist.

24. Verwendung nach irgendeinem der Ansprüche 22 und 23, wobei das partikuläre Material Bikarbonat oder Natriumchlorid beinhaltet.

25. Verwendung nach irgendeinem der Ansprüche 22 bis 24, wobei die Vorrichtung die Merkmale von irgendeinem der Ansprüche 2 bis 19 einschließt.

26. System zur Vorbereitung einer flüssigen Lösung für ein medizinisches Verfahren, wobei das System umfasst:
eine Kartusche (9), die ein partikuläres Material in einem Innenraum davon beinhaltet, und einen Einlass und einen Auslass umfasst,
eine erste Flüssigkeit (3)-Leitung mit einem ersten Ende (4), welches mit einer Quelle (1) der Flüssigkeit kommuniziert um die Flüssigkeit in die erste Flüssigkeitsleitung und ein zweites Ende zu entnehmen,
eine zweite Flüssigkeitsleitung (6) mit einem ersten Ende (7), welches mit der Quelle (1) der Flüssigkeit kommuniziert, und mit einem zweiten Ende (8), welches mit dem Einlass der Kartusche (9) zum Einführen der Flüssigkeit in den Innenraum (10) kommuniziert, um eine konzentrierte Flüssigkeitslösung herzustellen, welche zumindest einen Teil des partikulären in der Flüssigkeit gelösten Materials enthält, und
eine dritte Flüssigkeitsleitung (11), welche mit dem Auslass der Kartusche und mit einem Mischpunkt (13) in der ersten Flüssigkeitsleitung (3) zwischen den ersten und zweiten Enden (4, 5) kommuniziert zum Leiten der konzentrierten Flüssigkeitslösung von der Kartusche (9) in die ersten Flüssigkeitsleitung, um mit der Flüssigkeit gemischt zu werden, welche durch die erste Flüssigkeitsleitung geleitet wird, und dabei eine flüssige Lösung in der ersten Flüssigkeitsleitung zur Zuführung zu dem zweiten Ende der ersten Flüssigkeitsleitung herzustellen, wobei die Kartusche eine Vorrichtung wie in irgendeinem der Ansprüche 1 bis 19 definiert umfasst.

27. System nach Anspruch 26, wobei die Vorrichtung die Merkmale von irgendeinem der Ansprüche 2 bis 19 einschließt.

28. System nach irgendeinem der Ansprüche 26 und 27, wobei die Kartusche (9) einen Filter (23) einschließt, welcher bei dem Auslass (22) angeordnet ist, um einen Durchgang der Flüssigkeit durch den Filter (23) zu erlauben, jedoch um einen Durchgang des partikulären Materials durch den Filter zu verhindern, wobei der Filter der Flüssigkeit erlaubt, durch den Filter in einer Filterrichtung (a) hindurch zu gehen.

29. System nach Anspruch 28, wobei der Filter zumindest eine schlitzförmige Öffnung (30) umfasst, welche eine erste Ausdehnung (31) und eine zweite Ausdehnung (32) aufweist, welche im Wesentlichen senkrecht zu der Filterrichtung (a) und zu der ersten Ausdehnung ist, wobei die zweite (32) Ausdehnung deutlich kürzer ist, als die erste Ausdehnung (31).

30. System nach irgendeinem der Ansprüche 26 bis 29, wobei die Flüssigkeit eine Dialyseflüssigkeit ist.

31. System nach irgendeinem der Ansprüche 26 bis 30, wobei das partikuläre Material Bikarbonat und/oder Natriumchlorid einschließt.

## Revendications

1. Cartouche pour préparer une solution liquide pour une procédure médicale et agencée pour contenir un matériau en particules (20), ladite cartouche (9) incluant :
un espace intérieur (10) pour abriter le matériau en particules (20) ;
une entrée (21) agencée pour permettre l'introduction d'un liquide dans l'espace intérieur (10) ;
une sortie (22) agencée pour permettre la décharge de liquide hors de l'espace intérieur (10) ; et
un dispositif comprenant un corps creux (24) défini par une paroi (50) qui enferme une cavité du corps, le corps ayant un axe central (x), une première extrémité (52) et une seconde extrémité (53), ladite première extrémité étant montée sur la cartouche (9) à l'entrée d'une manière telle que le corps (24) s'étend dans l'espace intérieur (10) et ladite seconde extrémité étant située dans l'espace intérieur de la cartouche, ladite première extrémité étant ouverte et adaptée à recevoir ledit liquide à introduire dans la cartouche,
dans laquelle le dispositif inclut une pluralité d'ouvertures en forme de fentes (55), qui s'étendent à travers la paroi (50), de sorte que le liquide quitte le dispositif à travers lesdites ouvertures en forme de fentes (55) dans une direction d'écoulement (b), lesdites ouvertures en forme de fentes ayant une première extension (61) et une seconde extension (62) qui est sensiblement perpendiculaire à la direction d'écoulement (b) et à la première extension (61), dans laquelle la seconde extension (62) est significativement plus courte que la première extension (61) et significativement plus courte que la longueur (63) des ouvertures en forme de fentes (55) dans la direction d'écoulement (b).

2. Cartouche selon la revendication 1, dans laquelle ledit corps creux (24) a une forme tubulaire allongée le long de l'axe central (x).

3. Cartouche selon l'une quelconque des revendications 1 et 2, dans laquelle ledit corps creux (24) est effilé le long de l'axe central (x) vers la seconde extrémité (53).

4. Cartouche selon l'une quelconque des revendications précédentes, dans laquelle ledit corps creux (24) à la première extrémité (52) possède des moyens d'engagement (73, 74) adaptés pour la connexion du dispositif à la cartouche. (9).

5. Cartouche selon l'une quelconque des revendications précédentes, dans laquelle la première extension (61) est sensiblement perpendiculaire à la direction d'écoulement (b).

6. Cartouche selon l'une quelconque des revendications précédentes, dans laquelle la seconde extension (62) est égale ou inférieure à 0,1 mm.

7. Cartouche selon l'une quelconque des revendications précédentes, dans laquelle la seconde extension (62) est égale ou inférieure à 0,08 mm.

8. Cartouche selon l'une quelconque des revendications précédentes, dans laquelle la seconde extension (62) est égale ou supérieure à 0,02 mm.

9. Cartouche selon l'une quelconque des revendications précédentes, dans laquelle la seconde extension (62) est égale ou supérieure à 0,04 mm.

10. Cartouche selon l'une quelconque des revendications précédentes, dans laquelle la seconde extension (62) a approximativement 0,06 mm.

11. Cartouche selon l'une quelconque des revendications précédentes, dans laquelle lesdites ouvertures en forme de fentes (55) sont distribuées autour de la paroi (50).

12. Cartouche selon l'une quelconque des revendications précédentes, dans laquelle ledit corps creux (24) possède une portion de paroi (81-84) au moins à proximité de la seconde extrémité (53), et dans lequel ladite ouverture en forme de fente (55) s'étend à travers ladite portion de paroi (81-84).

13. Cartouche selon la revendication 12, dans laquelle ladite portion de paroi (82) présente une forme semblable à un embout.

14. Cartouche selon la revendication 13, dans laquelle ladite portion de paroi (82) est sensiblement conique.

15. Cartouche selon la revendication 14, dans laquelle ladite portion de paroi (83, 84) est sensiblement plane.

16. Cartouche selon la revendication 15, dans laquelle une direction normale à ladite portion de paroi (83) forme un angle d'inclinaison vis-à-vis de l'axe central (x).

17. Cartouche selon l'une quelconque des revendications précédentes, dans laquelle la direction d'écoulement (b) forme un angle vis-à-vis de l'axe central (x).

18. Cartouche selon la revendication 17, dans laquelle l'axe central (x) s'étend sensiblement parallèlement à une direction normale de la portion de paroi plane (84).

19. Cartouche selon l'une quelconque des revendications précédentes, dans laquelle l'ouverture en forme de fente (55) possède une extrémité amont et une extrémité aval par rapport à la direction d'écoulement (b), dans laquelle la seconde extension de l'ouverture en forme de fente augmente dans la direction d'écoulement (b) depuis une valeur minimum à l'extrémité amont de l'ouverture en forme de fente jusqu'à une valeur maximum à l'extrémité aval de l'ouverture (55).

20. Cartouche selon l'une quelconque des revendications 1 à 19, dans laquelle la cartouche (9) inclut un filtre (23) agencé à la sortie (22) et permettant le passage de liquide à travers le filtre (23) mais empêchant le passage du matériau en particules à travers le filtre, dans laquelle le filtre permet au liquide de passer à travers le filtre dans une direction de filtrage (a).

21. Cartouche selon la revendication 20, dans laquelle le filtre (23) inclut au moins une ouverture en forme de fente (30), qui possède une première extension (31) et une seconde extension (32) qui est sensiblement perpendiculaire à la direction de filtrage (a) et à la première extension, et dans laquelle la seconde extension est significativement plus courte que la première extension.

22. Utilisation d'un dispositif dans une cartouche (9) selon l'une quelconque des revendications 1 à 21, pour préparer une solution liquide pour une procédure médicale et agencée pour contenir un matériau en particules (20),
l'utilisation incluant l'étape consistant à alimenter ledit liquide vers la cartouche (9) via l'entrée (21) d'une manière telle que le liquide passe à travers le matériau en particules (20) et de ce fait dissout au moins une partie du matériau en particules pour former une solution liquide.

23. Utilisation selon la revendication 22, dans laquelle le liquide est un liquide de dialyse.

24. Utilisation selon l'une quelconque des revendications 22 et 23, dans laquelle le matériau en particules inclut du bicarbonate et/ou du chlorure de sodium.

25. Utilisation selon l'une quelconque des revendications 22 à 24, dans lequel le dispositif inclut les caractéristiques de l'une quelconque des revendications 2 à 19.

26. Système pour préparer une solution liquide pour une procédure médicale, le système incluant :
une cartouche (9) contenant un matériau en particules dans un espace intérieur de celle-ci, et incluant une entrée et une sortie ;
un premier conduit à liquide (3) ayant une première extrémité (4) en communication avec une source (1) de liquide pour extraire le liquide vers le premier conduit à liquide, et une seconde extrémité ;
un second conduit à liquide (6) ayant une première extrémité (7) en communication avec la source (1) de liquide et une seconde extrémité (8) en communication avec l'entrée de la cartouche (9) pour introduire le liquide dans l'espace intérieur (10) et produire une solution liquide concentrée contenant au moins une partie du matériau en particules dissous dans le liquide ; et un troisième conduit à liquide (11) en communication avec la sortie de la cartouche et avec un point de mélange (13) dans le premier conduit à liquide (3) en position intermédiaire entre ladite première et ladite seconde extrémité (4, 5) pour mener ladite solution liquide concentrée depuis la cartouche (9) jusque dans ledit premier conduit à liquide pour être mélangé avec le liquide qui est mené à travers le premier conduit à liquide et produire ainsi ladite solution liquide dans le premier conduit à liquide pour sa distribution vers la seconde extrémité du premier conduit à liquide, la cartouche comprenant un dispositif tel que défini dans l'une quelconque des revendications 1 à 19.

27. Système selon la revendication 26, dans lequel le dispositif inclut les caractéristiques de l'une quelconque des revendications 2 à 19.

28. Système selon l'une quelconque des revendications 26 et 27, dans lequel la cartouche (9) inclut un filtre (23) agencé à la sortie (22) et permettant le passage du liquide à travers le filtre (23) mais empêchant le passage du matériau en particules à travers le filtre, dans lequel le filtre permet au liquide de passer à travers le filtre dans une direction de filtrage (a).

29. Système selon la revendication 28, dans lequel le filtre inclut au moins une ouverture en forme de fente (30), qui possède une première extension (31) et une seconde extension (32) qui est sensiblement perpendiculaire à la direction de filtrage (a) et à la première extension, dans lequel la seconde extension (32) est significativement plus courte que la première extension (31).

30. Système selon l'une quelconque des revendications 26 à 29, dans lequel le liquide est un liquide de dialyse.

31. Système selon l'une quelconque des revendications 26 à 30, dans lequel le matériau en particules inclut du bicarbonate et/ou du chlorure de sodium.
